# EUROPÄISCHE PATENTANMELDUNG

(11) **EP 2 060 241 A1**
(43) Veröffentlichungstag der Anmeldung: **20.05.2009**
(21) Anmeldenummer: 08016609.3
(22) Anmeldetag: 22.09.2008
(51) Int. Cl.: A61C 8/00, A61B 17/88, B25B 23/10

(54) **Haltevorrichtung für Implantatteile**

(30) Priorität: 15.11.2007 DE 102007054894
(71) Anmelder: Heraeus Kulzer GmbH, 63450 Hanau (DE)
(72) Erfinder: Winterhoff, Jörg, 63450 Hanau (DE)
(74) Vertreter: Kühn, Hans-Christian

(57) **Zusammenfassung**

Die Erfindung betrifft eine Vorrichtung zum Halten von Teilen, insbesondere von Dentalimplantatteilen, mit einem Aufnahmekörper, der einen Hohlraum aufweist, wobei der Hohlraum durch eine Austrittsöffnung mit der Außenseite des Aufnahmekörpers verbunden ist, und besteht darin, dass um die Austrittsöffnung herum eine erste Anschlagfläche ausgebildet ist, dass in dem Hohlraum eine in Richtung ihrer Längsachse beweglich angeordnete Zugstange angeordnet ist, die durch die Austrittsöffnung aus dem Hohlraum herausragt, dass an dem aus dem Hohlraum herausragenden Bereich der Zugstange eine der ersten Anschlagfläche zugewandte zweite Anschlagfläche beabstandet von der ersten Anschlagfläche angeordnet ist und dass zwischen der ersten und der zweiten Anschlagfläche ein elastischer Ring um die Zugstange herum angeordnet ist.

## Beschreibung

Die Erfindung betrifft eine Vorrichtung zum Halten von Teilen, insbesondere zum Halten von Dentalimplantatteilen, mit einem Aufnahmekörper, der einen Hohlraum aufweist, wobei der Hohlraum durch eine Austrittsöffnung mit der Außenseite des Aufnahmekörpers verbunden ist.

Vielfach müssen mechanisch relativ empfindliche Teile, beispielsweise für eine Bearbeitung, sicher gehalten werden können. Zum Beispiel Implantataufbauteile, die auch aus Keramik bestehen können, müssen während des Beschleifens durch den Zahntechniker, sicher gehalten werden. Da die Teile überwiegend an ihrer Außenseite bearbeitet werden, muss das Halten über die Innenkonturen der Teile erfolgen. Dabei dürfen sie durch die beim Beschleifvorgang entstehenden Kräfte und Vibrationen nicht beschädigt werden. Die Halterung muss einfach zu bedienen sein und die gestellten Anforderungen im Übrigen sicher erfüllen. Anderenfalls ist eine genaue Bearbeitung nicht möglich und die Teile, insbesondere wenn sie aus Keramik bestehen, können beschädigt werden.

Der Erfindung liegt die Aufgabe zugrunde, auf dem Markt befindliche Teile zu verbessern und ein sicheres und beschädigungsfreies Halten von kleineren Teilen, insbesondere von Implantatteilen beim Bearbeiten, insbesondere beim Beschleifen, zu ermöglichen.

Die Aufgabe wird durch die Merkmale des Anspruchs 1 gelöst. Vorteilhafte Ausgestaltungen sind in den Unteransprüchen angegeben. Insbesondere dadurch, dass um die Austrittsöffnung herum eine erste Anschlagfläche ausgebildet ist, dass in dem Hohlraum eine in Richtung ihrer Längsachse beweglich angeordnete Zugstange angeordnet ist, die durch die Austrittsöffnung aus dem Hohlraum herausragt, dass an dem aus dem Hohlraum herausragenden Bereich der Zugstange eine der ersten Anschlagfläche zugewandte zweite Anschlagfläche beabstandet von der ersten Anschlagfläche angeordnet ist und dass zwischen der ersten und der zweiten Anschlagfläche ein elastischer Ring um die Zugstange herum angeordnet ist, wird eine beschädigungsfreie Halterung empfindlicher Teile ermöglicht. Solche Teile, wie sie beispielsweise keramische Implantataufbauteile darstellen, werden über den elastischen Ring der Vorrichtung gesteckt. Durch ein aufeinander zu Bewegen der beiden Anschlagflächen wird der Ring in seiner axialen Richtung gequetscht, wobei sich sein Durchmesser vergrößert. Durch diese Durchmesservergrößerung drückt sich der Ring gegen die Innenkontur des zu haltenden Teils und fixiert dieses an der Vorrichtung. Dazu ist es vorteilhaft, wenn der innere Durchmesser des elastischen Ringes genauso groß oder nur unwesentlich größer ist als der Durchmesser der Zugstange im Bereich des elastischen Ringes, da dadurch die radiale Ausdehnung des Ringes praktisch vollständig nach außen gerichtet ist. Dazu ist es insbesondere zweckmäßig, dass die Zugstange an einem Teil ihrer Länge ein Gewinde aufweist und mittels des Gewindes beweglich in dem Aufnahmekörper angeordnet ist, so dass sie praktisch in den Aufnahmekörper hineingeschraubt werden kann, wodurch sich die zweite Anschlagfläche auf die erste Anschlagfläche zu bewegt.

An der Zugstange und dem Aufnahmekörper kann ein Verdrehschutz angeordnet sein, der verhindert, dass sich der aus dem Aufnahmekörper herausragende Teil der Zugstange beim Ein- oder Ausschrauben in dem Aufnahmekörper mitdreht. Dadurch wird der Spann- bzw. Entspannvorgang erleichtert. Der Verdrehschutz zwischen Aufnahmekörper und Zugstange kann beispielsweise als Feder-Nut-Verbindung ausgeführt sein, wobei eine an einem der beiden Teile angeordnete Feder in eine an dem anderen der beiden Teile in dessen Längsrichtung angeordnete Nut eingreift, so dass eine Längsbewegung möglich ist und eine Drehbewegung verhindert wird. Es können mehrere Feder-Nut-Verbindungen um den Umfang der Zugstange herum angeordnet sein.

Das Gewinde greift vorteilhafterweise in ein mit dem Aufnahmekörper beweglich verbundenes Gegengewinde ein, wobei das Gegengewinde mittels eines Griffstücks um seine Längsachse herum drehbar ist. Das Gegengewinde kann ein Teil des Griffstückes sein, so dass das Griffstück praktisch als Schraubenmutter ausgebildet ist. Das Griffstück kann an einer der ersten Stirnseite gegenüber liegenden Seite des Aufnahmekörpers angeordnet sein, so dass die Schraubbewegung nahezu vollständig in eine Längsbewegung der zweiten Anschlagfläche der Zugstange umgesetzt wird. Das Griffstück kann dabei auch von der Zugstange abgeschraubt werden, so dass das Innere des Aufnahmekörpers zugänglich wird. Das Innengewinde des Griffstückes kann insbesondere in einem sogenannten Sackloch angeordnet sein, so dass die Bewegung und damit die Quetschung des elastischen Ringes begrenzt werden kann, da die Bewegung des Außengewindes der Zugstange begrenzt wird.

Die Zugstange kann aus mehreren Teilen gebildet sein, die vorzugsweise miteinander verschraubt sind. Dadurch ist eine leichte Montage und Demontage möglich. Insbesondere kann ein innerhalb des Aufnahmekörpers angeordneter Innenteil der Zugstange einen größeren Durchmesser als der durch die Austrittsöffnung herausragende Bereich der Zugstange aufweisen und es kann der Innenteil durch einen Austritt durch die Austrittsöffnung gehindert sein. Dies geschieht vorzugsweise durch eine Durchmesserverringerung des Inneren des Aufnahmekörpers. So kann beispielsweise die Austrittsöffnung einen kleineren Durchmesser aufweisen als der Innenteil der Zugstange. Der Innenteil der Zugstange und der aus dem Aufnahmekörper herausragende Teil der Zugstange können dabei miteinander verschraubt sein. Auf diese Weise ist ein leichtes Auseinandernehmen der Vorrichtung möglich, so dass z. B. der elastische Ring bei Bedarf ausgewechselt werden kann. Der Ring kann einen kreisförmigen äußeren Umfang oder auch einen Umfang in Form eines Vielecks aufweisen, so dass es möglich ist, die Außenkontur des Ringes an die Innenkontur des zu haltenden Teiles anzupassen, um ggf. die Verdrehsicherheit des gehaltenen Teils zu erhöhen. Zwar ist durch die Elastizität des Ringes ein Anpassen an unterschiedliche Innenkonturen bei den zu haltenden Teilen möglich, wenn jedoch häufig Teile mit gleicher Innenkontur gehalten werden müssen, kann eine Anpassung der Form des elastischen Ringes an diese Innenkontur vorteilhaft sein. Die Vorrichtung lässt sich also insbesondere auch zum Halten von Teilen mit einer Öffnung, die einen nicht kreisförmigen Querschnitt aufweist, verwenden. Insbesondere lässt sich die Vorrichtung zum Halten von Keramikteilen verwenden.

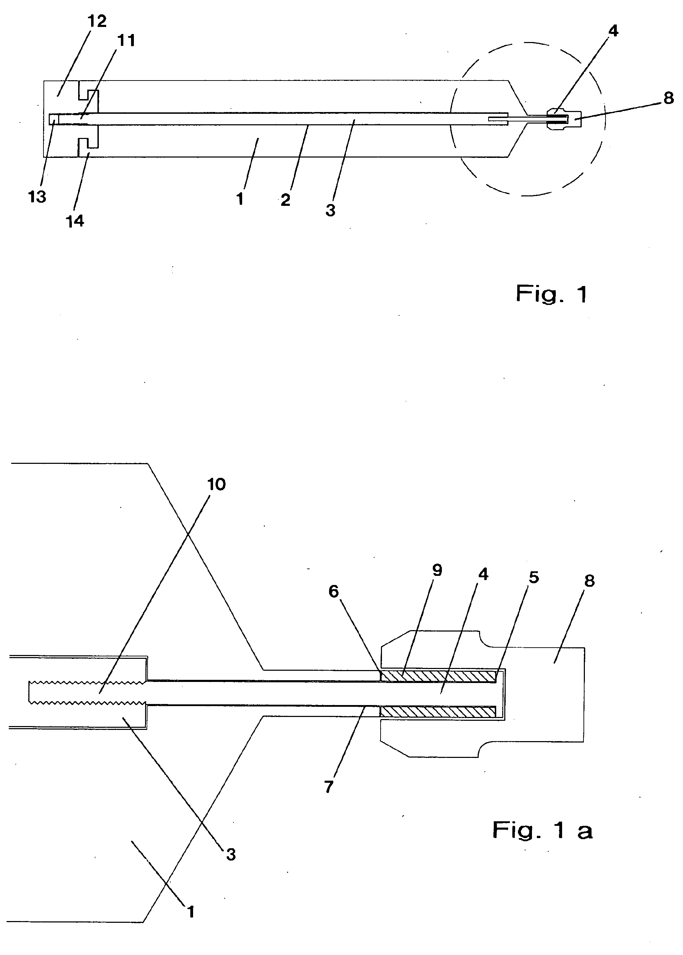

Nachfolgend wird ein Ausführungsbeispiel der Erfindung anhand einer Zeichnung erläutert. In der Zeichnung zeigt
- Figur 1: die schematische Darstellung einer erfindungsgemäßen Vorrichtung im gelösten Zustand
- Figur 1 a: eine Teilansicht aus Figur 1 mit Implantataufbauteil
- Figur 2: die Vorrichtung nach Figur 1, im gespannten Zustand
- Figur 2a: eine Teilansicht aus Figur 2 mit fixiertem Implantataufbauteil
- Figur 3a - 3c: verschiedene Querschnittsformen des elastischen Ringes.

Die erfindungsgemäße Vorrichtung weist in dem dargestellten Beispiel einen im Wesentlichen zylinderförmigen Aufnahmekörper 1 auf. In dem Hohlraum 2 des Aufnahmekörpers 1 ist der Innenteil 3 der Zugstange angeordnet, deren vorderer, aus dem Hohlraum 2 herausragender Bereich 4 an seinem Ende eine erste Anschlagfläche 5 aufweist. Diese ist einer zweiten Anschlagfläche 6 gegenüberliegend angeordnet, die die Austrittsöffnung 7 des Aufnahmekörpers 1 begrenzt. Die Austrittsöffnung 7 wird im gezeigten Beispiel durch einen zylindrischen Teil gebildet, der einen wesentlich geringeren Durchmesser als der Aufnahmekörper 1 aufweist. Dieser Durchmesser ist dem Innendurchmesser der zu haltenden keramischen Implantataufbauteile 8 angenähert, so dass der Aufnahmekörper eine für die Handhabung geeignete Größe aufweist bei gleichzeitiger Anpassung an die Größe der zu haltenden Teile, durch die verkleinerte "Aufnahmespitze", die einen elastischen Ring 9, z.B. aus Gummi aufweist. Der aus dem Hohlraum herausragende Bereich 4 der Zugstange weist einen Durchmesser von etwa 0,8 bis 1,5 mm auf. Sein inneres Ende 10 ist mit dem Innenteil 3 der Zugstange verschraubt. Dadurch ist zum einen eine einfache Montage der Vorrichtung gewährleistet, zum anderen kann der aus dem Hohlraum herausragende Bereich 4 der Zugstange leicht gelöst werden, so dass der elastische Ring 9 ersetzt werden kann. Dadurch ist unter anderem eine einfache Anpassung an die Innenkontur und den Innendurchmesser des zu haltenden Teils 8 möglich.

An dem aus dem Hohlraum 2 herausragenden und durch die Austrittsöffnung 7 geführten Teil der Zugstange ist eine in der Zeichnung nicht dargestellte Feder angeordnet, die in eine entsprechende, ebenfalls in der Zeichnung nicht dargestellte Nut in der Innenwand der Austrittsöffnung 7 eingreift, wobei sich die Nut in Längsrichtung des Aufnahmekörpers 1 erstreckt, so dass eine Längsbewegung der Zugstange möglich ist. Die Feder-Nut-Verbindung kann in an sich bekannter Weise ausgebildet sein. Die Feder kann länglich oder auch annähernd punktförmig ausgebildet sein.

Das dem herausragenden Bereich 4 abgewandte Ende 11 der Zugstange ist mittels eines Gewindes in dem Griffstück 12 gehalten. Das Griffstück 12 weist ein Sackloch 13 auf, so dass eine axiale Bewegung der Zugstange und damit eine Veränderung des Durchmessers des elastischen Ringes 9 zum Halten bzw. Lösen des zu haltenden Teiles 8 möglich ist. Das Griffstück 12 ist beispielsweise mittels einer Clipverbindung 14 (nach Art einer Feder-Nut-Verbindung) an dem Aufnahmekörper 1 drehbar fixiert.

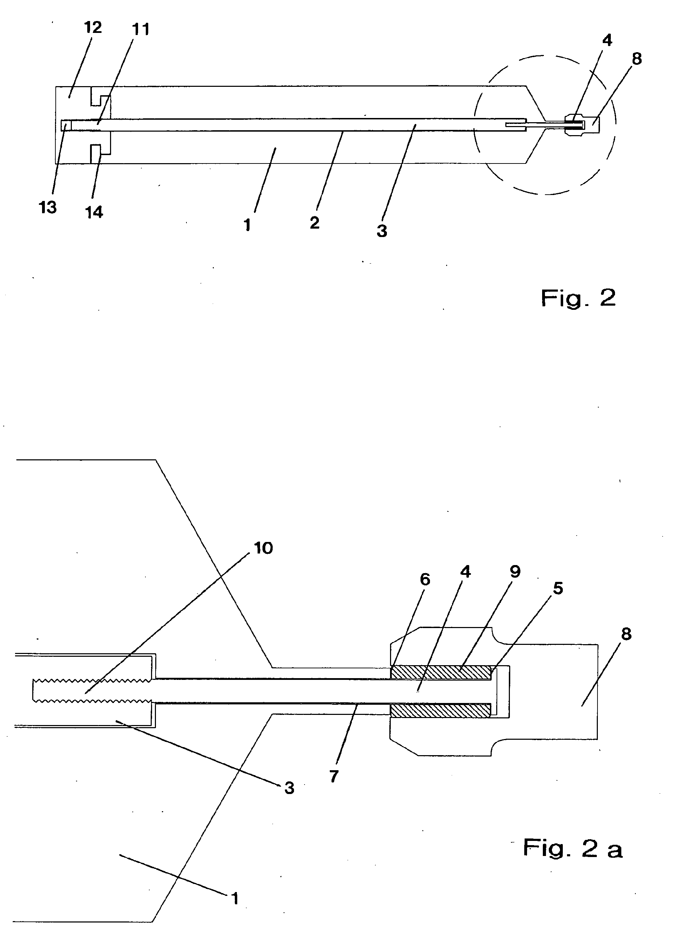

Figur 2 bzw. Figur 2 a zeigt ein auf der Haltevorrichtung fixiertes Teil 8. Der elastische Ring 9 ist durch Einschrauben der Zugstange in den Aufnahmekörper 1 in seiner Länge zusammengepresst und damit in seinem Durchmesser aufgeweitet. Er drückt gegen die Innenwand des Teils 8 und fixiert dieses.

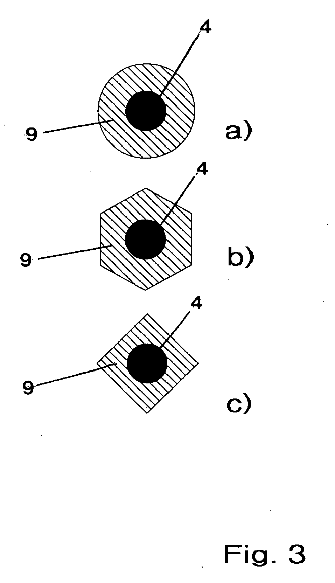

In der Figur 3 ist der Querschnitt eines elastischen Ringes 9 auf dem aus dem Hohlraum 2 herausragenden Bereich 4 der Zugstange dargestellt. Dabei ist der elastische Ring 9 einmal mit einem kreisförmigen, des Weiteren mit einem sechseckigen und schließlich mit einem quadratischen Querschnitt dargestellt. Damit oder mit unterschiedlichem Durchmesser des elastischen Ringes 9 ist eine optimale Anpassung an die verschiedensten zu haltenden Teile 8 möglich.

## Patentansprüche

1. Vorrichtung zum Halten von Teilen, insbesondere von Dentalimplantatteilen, mit einem Aufnahmekörper, der einen Hohlraum aufweist, wobei der Hohlraum durch eine Austrittsöffnung mit der Außenseite des Aufnahmekörpers verbunden ist, **dadurch gekennzeichnet, dass** um die Austrittsöffnung herum eine erste Anschlagfläche ausgebildet ist, dass in dem Hohlraum eine in Richtung ihrer Längsachse beweglich angeordnete Zugstange angeordnet ist, die durch die Austrittsöffnung aus dem Hohlraum herausragt, dass an dem aus dem Hohlraum herausragenden Bereich der Zugstange eine der ersten Anschlagfläche zugewandte zweite Anschlagfläche beabstandet von der ersten Anschlagfläche angeordnet ist und dass zwischen der ersten und der zweiten Anschlagfläche ein elastischer Ring um die Zugstange herum angeordnet ist.

2. Vorrichtung nach Anspruch 1, **dadurch gekennzeichnet, dass** die Zugstange an einem Teil ihrer Länge ein Gewinde aufweist und mittels des Gewindes beweglich in dem Aufnahmekörper angeordnet ist.

3. Vorrichtung nach Anspruch 2, **dadurch gekennzeichnet, dass** das Gewinde in ein mit dem Aufnahmekörper beweglich verbundenes Gegengewinde eingreift, wobei das Gegengewinde mittels eines Griffstücks um seine Längsachse herum drehbar ist.

4. Vorrichtung nach Anspruch 3, **dadurch gekennzeichnet, dass** das Griffstück an einer der ersten Stirnseite gegenüberliegenden Seite des Aufnahmekörpers angeordnet ist.

5. Vorrichtung nach Anspruch 3 oder 4, **dadurch gekennzeichnet, dass** die Zugstange ein Außengewinde aufweist, das in ein Innengewinde des Griffstücks eingreift.

6. Vorrichtung nach einem der Ansprüche 1 bis 5, **dadurch gekennzeichnet, dass** die Zugstange aus mehreren Teilen gebildet ist, die vorzugsweise miteinander verschraubt sind.

7. Vorrichtung nach Anspruch 6, **dadurch gekennzeichnet, dass** ein innerhalb des Aufnahmekörpers angeordneter Innenteil der Zugstange einen größeren Durchmesser als der durch die Austrittsöffnung herausragenden Bereich der Zugstange aufweist und dass der Innenteil, vorzugsweise durch eine Durchmesserverringerung des Innern des Aufnahmekörpers, an einem Austritt durch die Austrittsöffnung gehindert ist.

8. Verwendung einer Vorrichtung nach einem der Ansprüche 1 bis 5 zum Halten von Teilen mit einer Öffnung, die einen nicht kreisförmigem Querschnitt aufweist.

9. Verwendung einer Vorrichtung nach einem der Ansprüche 1 bis 5 zum Halten von Keramikteilen.
